# EUROPÄISCHE PATENTSCHRIFT

(11) **EP 0 511 727 B1**
(45) Veröffentlichungstag und Bekanntmachung des Hinweises auf die Patenterteilung: **15.11.1995**
(21) Anmeldenummer: 92250104.4
(22) Anmeldetag: 30.04.1992
(51) Int. Cl.: C07J 53/00, A61K 31/565

(54) **14Alpha,17alpha-(Propano- und 17,2-Propeno)-estratriene**
14-alpha,17alpha-(propano- and 17,2-propeno)-estratrienes
14-alpha,17alpha-(propano- et 17,2-propeno)-estratriènes

(30) Priorität: 30.04.1991 DE 4114634
(43) Veröffentlichungstag der Anmeldung: 04.11.1992
(73) Patentinhaber: SCHERING AKTIENGESELLSCHAFT, 13342 Berlin (DE)
(72) Erfinder: Bull, James R., Prof., Waterkloof Ridge 2 (ZA); Elger, Walter, Dr., W-1000 Berlin 33 (DE); Fritzemeier, Karl-Heinrich, Dr., W-1000 Berlin 27 (DE); Krattenmacher, Rolf, Dr., W-1000 Berlin 44 (DE)

(56) Entgegenhaltungen:
- EP-A- 0 372 665
- WO-A-88/01275
- DE-A- 3 808 679

## Beschreibung

Die Erfindung betrifft neue 14α,17α-(Propano- und 17²-Propeno)estratriene der allgemeinen Formel I 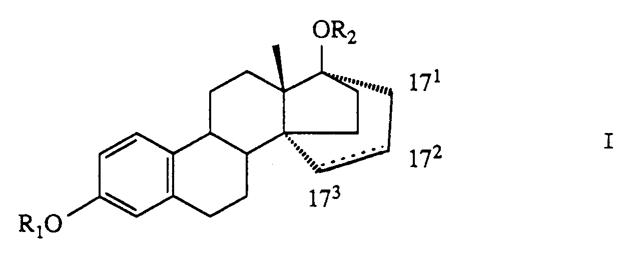
worin
R¹ ein Wasserstoffatom, eine Methyl- oder eine Acylgruppe mit 1-12 Kohlenstoffatomen,
R² ein Wasserstoffatom oder eine Acylgruppe mit 1-12 Kohlenstoffatomen und 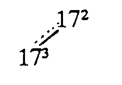
eine C-C-Einfach- oder Doppelbindung
bedeuten.
Als Acylgruppen R¹ und R² kommen Reste von organischen Carbonsäuren mit 1-12 Kohlenstoffatomen infrage. Sie leiten sich ab von aliphatischen, cycloaliphatischen, aliphatisch-cycloaliphatischen und aromatischen Monocarbonsäuren. Die Anzahl der Kohlenstoffatome im Ring variiert von 3 bis 5. Bevorzugt werden als Reste R¹ und R² die Acylgruppen der Essig-, Propion-, Butter-, Isobutter-, Pivalin-, Capron-, Heptyl-, Capryl-, Pelargon-, Decan-, Undecan-, Dodecan-, 3-Cyclopentylpropion- und Benzoesäure.
Aus der internationalen Patentanmeldung WO 88/01275 sind 14,17-Ethano-estratriene bekannt. Obwohl diese Verbindungen keine 17α-Ethinylgruppe enthalten, sind sie auch nach oraler Applikation östrogen wirksam wie Ethinylestradiol. Bislang war man der Meinung, daß zur Erzielung einer oralen Wirksamkeit die 17α-Ethinylgruppe notwendig ist.

Es wurde nun gefunden, daß die neuen 14α,17α-(Propano- und 17²-Propeno)estratriene ebenfalls nach oraler Applikation wirksam sind und dabei die Wirksamkeit von Ethinylestradiol übertreffen, wie aus der Tabelle 1 hervorgeht. Auch die Verbindungen der vorliegenden Anmeldung enthalten keine 17α-Ethinylgruppe.

Im Allen-Doisy-Test wird eine Bewertung von Vaginalabstrichen bei ovariektomierten Ratten an den Tagen 3 - 5 (d3-d5) nach der einmaligen Applikation am Tag 1 (d1) der Prüfsubstanz vorgenommen. Folgende Zyklusstadien werden unterschieden:
1 = Diöstrus (Leukozyten und kernhaltige Epithelzellen),
2 = Proöstrus (kernhaltige Epithelzellen)
3 = Östrus (kernlose Hornschollen)
4 = Metöstrus (kernlose Hornschollen, Leukozyten, Epithelzellen).
Estrogen wirksame Substanzen führen nach oraler oder subcutaner Applikation zur Proliferation des Vaginalepithels und zur Verhornung der oberflächlichen Zell-Lagen. Als Schwellenwert wird diejenige Menge eines Estrogens angesehen, bei der 50% der Tiere Stadium 3 erreichen. Außerdem bewirken Estrogene eine Zunahme des Uterusgewichts.

Die erfindungsgemäßen Verbindungen können in der gleichen Weise wie Ethinylestradiol formuliert und eingesetzt werden. Sie werden mit den in der galenischen Pharmazie üblichen Zusätzen, Trägersubstanzen und Geschmackskorrigenzien nach an sich bekannten Methoden zu den üblichen Arzneimittelformen verarbeitet. Für die orale Applikation kommen insbesondere Tabletten, Dragees, Kapseln, Pillen, Suspensionen oder Lösungen infrage. Für die parenterale Applikation kommen insbesondere ölige Lösungen, wie zum Beispiel Sesamöl- oder Rizinusöllösungen, infrage, die gegebenenfalls zusätzlich noch ein Verdünnungmittel, wie zum Beispiel Benzylbenzoat oder Benzylalkohol, enthalten können.
Die Wirkstoffkonzentration in den pharmazeutischen Zusammensetzungen ist abhängig von der Applikationsform und dem Anwendungsgebiet. So können zum Beispiel Kapseln oder Tabletten zur Behandlung von Östrogenmangelerscheinungen 0,001 bis 0,05 mg Wirkstoff, ölige Lösungen zur intramuskulären Injektion pro 1 ml etwa 0,01 bis 0,1 mg Wirkstoff und Vaginalsalben etwa 0,1 bis 10 mg pro 100 ml Salbe enthalten. Zur Kontrazeption bei der Frau können die erfindungsgemäßen Östrogene in Kombination mit Gestagenen angewandt werden. Tabletten oder Dragees sollen vorzugsweise 0,003 bis 0,05 mg des erfindungsgemäßen Östrogens und 0,05 bis 0,5 mg eines Gestagens enthalten.
Die erfindungsgemäßen Verbindungen können bei Östrogenmangelerscheinungen der Frau, wie zum Beispiel Amenorrhoe, Dysmenorrhoe, Sterilität, Frigidität, Endometritis, Kolpitis und klimakterischen Beschwerden verwendet werden. Ferner können die Verbindungen als östrogene Komponente in Kombinationspräparaten mit Gestagenen zur Fertilitätskontrolle bei der Frau eingesetzt werden.
Die 14α,17α-(Propano- und 17²-Propeno)estratriene der allgemeinen Formel I 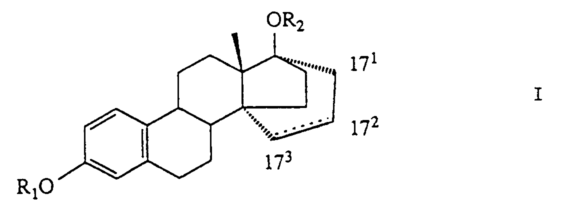
worin
R¹ ein Wasserstoffatom, eine Methyl- oder eine Acylgruppe mit 1-12 Kohlenstoffatomen,
R² ein Wasserstoffatom oder eine Acylgruppe mit 1-12 Kohlenstoffatomen und 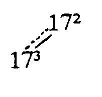
eine C-C-Einfachbindung oder Doppelbindung
bedeuten,
können hergestellt werden, indem man im Hydroxyketon der Formel II 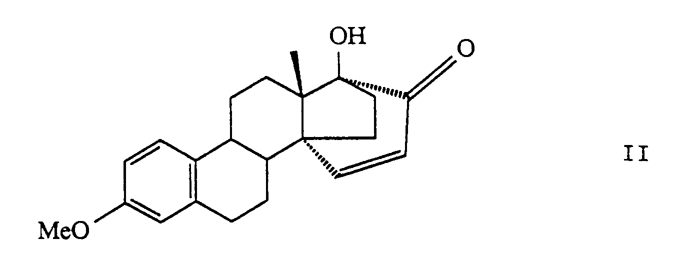
die Carbonylgruppe zur Methylengruppe reduziert, gegebenenfalls die 17²-17³-Doppelbindung hydriert, gegebenenfalls den 3-Methylether spaltet oder die 17-Hydroxygruppe verestert, gegebenenfalls die 3-Hydroxygruppe partiell oder die 3- und 17-Hydroxygruppen gleichzeitig verestert und gegebenenfalls eine so erhaltene 3,17-Diacyloxy- selektiv zur 3-Hydroxy-17-acyloxy-Verbindung verseift.

Die Reduktion der Carbonyl- zur Methylengruppe wird nach den in der Steroidchemie üblichen Methoden durchgeführt.
Nach einer bevorzugten Methode wird das Keton (Carbonyl) zunächst in das Dithioketal überführt und dann mit Natrium in flüssigem Ammoniak reduziert. Als Lösungsmittel kommen Tetrahydrofuran, Dioxan, Benzol und Toluol infrage.

Die Hydrierung der 17²-17³-Doppelbindung erfolgt in an sich bekannter Weise, vorzugsweise in Gegenwart eines Edelmetallkatalysators auf einem inerten Träger.

Die sich gegebenenfalls anschließende Spaltung eines 3-Methylethers wird nach den üblichen Methoden der Steroidetherspaltung vorgenommen. So kann die 3-Methyletherspaltung beispielsweise mit einer Lewissäure in einem inerten Lösungsmittel in der Siedehitze durchgeführt werden. Als Lewissäuren sind beispielsweise Bortrifluoridetherat oder Diisobutylaluminiumhydrid (DIBAH) geeignet. Als Lösungsmittel kommen Benzol, Toluol, Tetrahydrofuran, Dioxan u.a. infrage.

Die Verseifung der Acyloxygruppen kann in an sich bekannter Weise erfolgen. Beispielsweise wird die Verseifung mit Basen in wäßrig-alkoholischer Lösung, wie Kaliumhydroxid in wäßrig-methanolischer Lösung, vorgenommen.

Für die sich gegebenenfalls anschließende Veresterung der phenolischen und tertiären Hydroxygruppe kommen die üblicherweise in der Steroidchemie zur Veresterung angewendeten Verfahren infrage. Beispielsweise sei die Umsetzung mit Essigsäure oder Acetanhydrid in Gegenwart starker Säuren, wie zum Beispiel Trifluoressigsäure, Perchlorsäure oder p-Toluolsulfonsäure, bei Raumtemperatur oder etwas angehobener Temperatur oder die Umsetzung mit Acetanhydrid in Gegenwart eines tertiären Amins bei etwa 20-80°C genannt.

Werden Pyridin und 4-Dimethylamino-pyridin als tertiäre Amine gemeinsam angewandt, kann die Veresterung mit niederen Carbonsäuren vorzugsweise bei Raumtemperatur und mit höheren Carbonsäuren vorzugsweise bei 40-80°C durchgeführt werden.

Die Synthesen der beiden möglichen Halbester erfolgen durch partielle Veresterung oder partielle Verseifung:
a) Ausgehend von den 3,17β-Dihydroxyverbindungen lassen sich durch selektive Veresterung der phenolischen Hydroxygruppe die 3-Acyloxy-17β-hydroxyverbindungen erhalten. Die Reaktionen werden durch Umsetzungen des entsprechenden Säureanhydrids in Gegenwart eines heterocyclischen Stickstoffaromaten, vorzugsweise Pyridin, erreicht. Als Reaktionstemperatur eignet sich der Bereich zwischen Raum- und Siedetemperatur der Reaktionsmischung.
b) Ausgehend von den 3,17β-Diacyloxyverbindungen lassen sich durch selektive Verseifüng der phenolischen Acyloxygruppe die 3-Hydroxy-17α-acyloxyverbindungen erhal ten. Die Synthesen erfolgen durch Umsetzungen mit einem Alkalicarbonat oder Erdalkalicarbonat, vorzugsweise Kalium- oder Calciumcarbonat, in wäßrig-methanolischer Lösung. Als Reaktionstemperatur eignet sich der Bereich zwischen Raum- und Siedetemperatur der Reaktionsmischung.
Nachfolgend ist die Herstellung der Ausgangsverbindung (Hydroxyketone der Formel II - identisch mit Verbindung 11 -) beschrieben. Die sich daran anschließenden Beispiele dienen der näheren Erläuterung der Erfindung.

### Herstellung der Ausgangsverbindungen

### 3-Methoxy-16-methylestra-1,3,5(10),14,16-pentaen-17-yl acetat (2)

Zu einer Suspension von 3-Methoxy-16-methylestra-1,3,5(10),15-tetraen-17-on (**1**) (DE-OS 3.023.568 (1982)(20 g; 67,7 mmol) in Isopropenylacetat (400 ml) wird Essigsäureanhydrid (80 ml) und p-Toluolsulfonsäure (6 g) gegeben. Das Reaktionsgemisch wird bei 100°C ca 20 Stunden gerührt. Nach dem Abkühlen wird auf Eiswasser gegossen und durch Zugabe kleiner Portionen Natriumhydrogencarbonat neutralisiert. Man extrahiert dreimal mit Benzol, wäscht die vereinigten organischen Phasen mit Wasser, trocknet über Magnesiumsulfat und engt am Vakuum ein. Der erhaltene braune Rückstand (23,5 g) wird an Kieselgel mit einem Gemisch aus Ethylacetat/Benzol (1:49) chromatographiert Das gelblich kristalline Produkt wird durch Umkristallisation aus Ethylacetat/Methanol weiter aufgereinigt. Es werden 19,8 g (87%) farbloses 14,16-Dien-17-acetat (2) erhalten.
Fp.=129-130°C; [α]²⁰_{D}=+259° (CHCl₃; c=0,9)

### Cycloaddition von Phenylvinylsulfon an das Dienylacetat (2)

Eine Mischung des Dienylacetats (**2**) (3,38 g; 10 mmol) und Phenylvinylsulfon (1,76 g; 10,5 mmol) in absolutem Xylol (6 ml) wird in einem Druckgefäß unter Stickstoff 20 Stunden auf 140°C erhitzt. Die Dünnschichtchromatographie zeigt neben wenig Startmaterial die 3 Produkte (**3**), (**4**), (**5**) [R_{f}-Werte ca. 0,38; 0,36; 0,33; Laufmittel: Ethylacetat/Toluol (1:9)]. Nach dem Abkühlen fällt das Produkt mit dem mittleren R_{f}-Wert (0,36) aus. Der Rückstand wird abfiltriert und aus Chloroform/Benzol kristallisiert. Man erhält (17²R)-3-Methoxy-16-methyl-17²-(phenylsulfonyl)- 14α,17α-ethanoestra-1,3,5(10),15-te traen-17β-yl acetat (**4**) (720 mg; 14%).
Fp.=260-262°C; [α]²⁰_{D}=103° (CHCl₃; c=0,9)
Das Filtrat wird durch Flash-Chromatographie an Kieselgel mit einem Gemisch aus Ethylacetat/Benzol (1:19) gereinigt. Es wird neben verunreinigtem Ausgangsmaterial (**2**) (300 mg) ein Gemisch der Cycloaddukte (**3**) und (**5**) (3,8 g) im Verhältnis 1:1 erhalten. Ein Teil dieses Gemisches wird erneut an Kieselgel chromatographiert; man erhält sirupöses 3-Methoxy-17¹-methyl-16α-(phenylsulfonyl)-14α,17α-ethenoestra-1,3,5(10)-men-17β-yl acetat (**3**).
[α]²⁰_{D}=+61° (CHCl₃; c=0,1); IR (CHCl₃): 1735 cm⁻¹.

### Reduktive Desulfonierung der Cycloaddukte (3) und (5)

Ein Gemisch der Cycloaddukte (**3**) und (**5**) (ca 1:1) (22,26 g; 44 mmol) wird in einer Mischung aus absolutem Tetrahydrofuran (80 ml) und absolutem Methanol (320 ml) gelöst. Nach Zugabe von Natriumhydrogencarbonat wird das Reaktionsgemisch auf 0°C gekühlt. Unter kräftigem Rühren wird Natriumamalgam (6%ig; 83 g) hinzugesetzt. Nach 2 Stunden wird weiteres Natriumamalgam (29 g) zugegeben. Man läßt weitere 2 Stunden bei 0°C, sowie 16 Stunden bei 25°C nachrühren. Anschließend wird zur Zerstörung überschüssigen Reagenzes vorsichtig Wasser (50 ml) hinzugefügt und das Gemisch unter vermindertem Druck auf ca 1/3 des Volumens eingeengt. Man addiert weitere 300 ml Wasser und extrahiert mit Chloroform. Die organische Phase wird mit Wasser gewaschen und über Magnesiumsulfat getrocknet. Es wird am Vakuum eingeengt und der erhaltene Rückstand (13 g) durch Säulenchromatographie an Kieselgel mit einem Gemisch aus Ethylacetat/Benzol (1:9) gereinigt. Man erhält 3-Methoxy-17¹-methyl-14α,-17α-ethenoestra-1,3,5(10)-trien-17β-ol (**6**) (11,5 g; 81%).
Fp.=147-149°C (nach Kristallisation aus wäßrigem Methanol); [α]²⁰_{D}=129° (CHCl₃; c=0,8)

### 3-Methoxy-17¹-methyl-14α,17α-ethenoestra-1,3,5(10)-trien-17β-yl acetat (7)

Zu einer Suspension der Verbindung **6** (2 g; 6,2 mmol) in Essigsäureanhydrid (10 ml) wird bei 0°C unter Rühren p-Toluolsulfonsäure (200 mg) gegeben. Zunächst entsteht eine klare Lösung, aus der langsam ein Niederschlag ausfällt. Nach einer Stunde wird Wasser addiert, der Niederschlag abfiltriert und an Kieselgel mit Benzol als Laufmittel chromatographiert. Man erhält 2,06g (91%) der Verbindung (**7**).
Fp.=117-119°C (nach Kristallisation aus Dichlormethan/Methanol); [α]²⁰_{D}=+87° (CHCl₃; c=0,9).

### Hydroxylierung des Acetoxy-olefins (7)

Zu einer Lösung von Verbindung **7** (586 mg; 1,6 mmol) in absolutem Pyridin (10 ml) wird Osmiumtetroxid gegeben. Man läßt 48 Stunden bei 25°C nachrühren, kühlt auf 0°C und addiert wäßrige Natriumdisulfitlösung (10%ig; 20 ml). Es wird weitere 30 Minuten gerührt und dann mit Benzol extrahiert. Das so erhaltene Rohprodukt (690 mg) wird an Kieselgel mit einem Gemisch aus Ethylacetat/Benzol (1:7) chromatographiert. Man erhält (17¹S,17²S)-17¹,17²-Dihydroxy-3-methoxy-17¹-methyl-14α,17α-ethanoestra-1,3,5(10)-trien-17β-yl acetat (**8**) (527 mg; 82%).
Fp.=209-210°C (nach Kristallisation aus Benzol/Hexan); [α]²⁰_{D}=+16° (CHCl₃; c=0,85); IR (CHCl₃): 3600-3250 (OH) und 1710 (OAc) cm⁻¹.
Weitere Elution mit Ethylacetat/Benzol (1:4) ergibt (17¹R,17²R)-14α,17α-Ethano-17¹,17²-dihydroxy-3-methoxy-17¹-methylestra-1,3,5(10)-trien-17β-yl acetat (**9**) (45 mg; 7%).
Fp.=169-170°C (nach Kristallisation aus Benzol/Hexan); [α]²⁰_{D}=+31° (CHCl₃; c=0,9); IR(CHCl₃): 3690 und 3550-3150 (OH) und 1710 (OAc) cm⁻¹.

### 17β-(Acetoxy)-3-methoxy-20-oxo-19-nor-17α-pregna-1,3,5(10)-trien-14-carbaldehyd (10)

Zu einer Suspension des Diols (**8**) (100 mg; 0,25 mmol) in Ethanol (10 ml) wird bei 25°C unter Rühren eine wäßrige Natriumperiodatlösung (6%ig, 3 ml) hinzugefügt. Man läßt 6 Stunden bei 25°C nachrühren. Anschließend wird am Vakuum auf die Hälfte des Volumens eingeengt, mit Wasser versetzt und mit Chloroform extrahiert. Die organische Phase wird mit ges. Natriumchloridlösung gewaschen, über Magnesiumsulfat getrocknet und am Vakuum eingeengt. Das erhaltene Rohprodukt wird durch Kristallisation aus Ethylacetat gereinigt. Man erhält 94 mg der Verbindung **10**.
Fp.=212-213°C; [α]²⁰_{D}=+3,5° (CHCl₃; c=0,85); IR (CHCl₃): 1736 (OAc) und 1715 br. (14¹-und 20-C=O) cm⁻¹.

### 17β-Hydroxy-3-methoxy-14α,17α-(17²-propeno)estra-1,3,5(10)-trien-17¹-on (11)

Eine Lösung von Verbindung **10** (430 mg, 1,08 mmol) in 1 molarer methanolischer Kaliumhydroxidlösung (10 ml) wird 3 Stunden bei 25°C gerührt. Anschließend fügt man Wasser hinzu und extrahiert mit Toluol. Die organische Phase wird mit gesättigter Natriumchloridlösung gewaschen und über Magnesiumsulfat getrocknet. Man engt am Vakuum ein und reinigt das erhaltene Rohprodukt durch Kristallisation aus Chloroform/Methanol. Man erhält 114 mg des 17-Hydroxyketons (**11**).
Fp.=177-180°C; [α]²⁰_{D}=+195° (CHCl₃; c=0,8); IR (CHCl₃): 3460 und 1670 cm⁻¹.

Säulenchromatographie des Rückstandes der Mutterlauge an Kieselgel mit Ethylacetat/Toluol (1:9) als Laufmittel ergibt weitere 197 mg der Verbindung **11**.

### 3-Methoxy-14α,17α-(17¹-oxo-17²-propeno)estra-1,3,5(10)-trien-17β-yl acetat (13)

a) Der Oxoaldehyd **10** (250 mg; 0,63 mmol) wird mit konz. Salzsäure (1 ml) in Tetrahydrofuran (10 ml) 3 Stunden bei 50°C gerührt. Anschließend wird die Reaktionslösung mit wäßriger Natriumhydrogencarbonatlösung neutralisiert und mit Toluol extrahiert. Die organische Phase wird mit gesättigter Natriumchloridlösung gewaschen und über Magnesiumsulfat getrocknet. Man engt am Vakuum ein und reinigt das erhaltene Rohprodukt durch Säulenchromatographie an Kieselgel mit Ethylacetat/Toluol als Laufmittel. Man erhält 234 mg des Acetoxyketons **13**.
   Fp.=200-204°C (nach Kristallisation aus Chloroform/Methanol); [α:]²⁰_{D}=+174° (CHCl₃; c=1,0); IR (CHCl₃): 1739 und 1693 cm⁻¹.
b) Zu einer Lösung des Hydroxyketons **11** (150 mg) in Toluol (6 ml) wird bei 25°C Essigsäureanhydrid, sowie p-Toluolsulfonsäure gegeben. Man rührt 4 Stunden bei 25°C nach, neutralisiert dann mit wäßriger Natriumhydrogencarbonatlösung und extrahiert mit Toluol. Die organische Phase wird mit ges. Natriumhydrogencarbonatlösung und ges. Natriumchloridlösung gewaschen und über Magnesiumsulfat getrocknet. Es wird am Vakuum eingeengt und das erhaltene Rohprodukt durch Säulenchromatographie an Kieselgel mit einem Gemisch aus Ethylacetat/Toluol gereinigt. Man erhält 149 mg der Verbindung **13**.
   Fp.=200-204°C

### Beispiel 1

### 3-Methoxy-14α,17α-(17²-propeno)estra-1,3,5(10)-trien-17β-ol (15)

Verbindung **11** (466 mg; 1,4 mmol) wird in Eisessig (3 ml) gelöst. Man versetzt mit Ethandithiol (1 ml) und addiert dann Bortrifluorid-Diethylether-Komplex (120 µl) in 3 gleich großen Portionen über einen Zeitraum von 30 Stunden bei 30°C. Anschließend wird das Reaktionsgemisch auf wäßrige Natriumhydrogencarbonatlösung gegossen. Man extrahiert mit Toluol, wäscht die organische Phase mit gesättigter Natriumchloridlösung und trocknet über Magnesiumsulfat. Es wird am Vakuum eingeengt und das erhaltene Rohprodukt durch Säulenchromatographie an Kieselgel mit einem Gemisch aus Ethylacetat/Toluol (1:9) gereinigt. Man erhält 560 mg des Thioketals (**14**). IR: 3481 cm⁻¹ Verbindung **14** (350 mg; 0,85 mmol) wird in absolutem Tetrahydrofuran gelöst und unter Rühren zu einem Gemisch aus Natrium (195 mg) in flüssigem Ammoniak (60 ml) gegeben. Man rührt ca. 2 Stunden bei -33°C nach. Anschließend läßt man nach Zugabe von festem Ammoniumchlorid den Ammoniak abdampfen. Der Rückstand wird mit Wasser versetzt. Man extrahiert mit Toluol, wäscht die organische Phase mit gesättigter Natriumchloridlösung trocknet über Magnesiumsulfat und engt am Vakuum ein. Das erhaltene Rohprodukt wird durch Säulenchromatographie an Kieselgel mit einem Gemisch aus Ethylacetat/Toluol (1:19) gereinigt. Man erhält 230 mg der 14α,17α-Propeno-Verbindung **15**.
Fp.=141-142°C (nach Kristallisation aus Chloroform/Methanol); [α]²⁰_{D}=+137° (CHCl₃; c=0,1); IR (CHCl₃): 3603 cm⁻¹.

### Beispiel 2

### 14α,17α-(17²-Propeno)estra-1,3,5(10)-trien-3,17β-diol (16)

Zu einer Lösung der Verbindung **15** (110 mg; 0,34 mmol) in absolutem Toluol (5 ml) wird Diisobutylaluminiumhydrid (1 molar in Hexan; 3,5 ml; 3,5 mmol) hinzugegeben. Anschließend kocht man 24 Stunden unter Rückfluß. Nach Abkühlung auf Raumtemperatur wird mit verdünnter Salzsäure angesäuert. Man extrahiert mit Methanol/Chloroform (1:9), trocknet über Magnesiumsulfat und eng am Vakuum ein. Das so erhaltene Rohprodukt wird durch Säulenchromatographie an Kieselgel mit einem Gemisch aus Ethylacetat/Toluol (1:4) gereinigt. Man erhält 90 mg des Diols **16**.
Fp.= 156-159°C (nach Kristallisation aus Ethylacetat); [α]²⁰_{D}=+150° (c=0,9; Tetrahydrofuran); MS: m/e= 310 (M⁺).

### Beispiel 3

### 3-Methoxy-14α,17α-propanoestra-1,3,5(10)-trien-17β-ol (17)

Die Verbindung **15** (175 mg) wird in Ethylacetat gelöst und in Gegenwart von Palladium-Aktivkohle (10%ig; 40 mg) bei 1,3 atm. Wasserstoffdruck 30 Stunden lang hydriert Anschließend filtriert man ab und engt am Vakuum ein. Das so erhaltene Rohprodukt wird durch Säulenchromatographie an Kieselgel mit einem Gemisch aus Ethylacetat/Toluol gereinigt. Man erhält 170 mg der Verbindung **17**.
Fp.=155-157°C (nach Kristallisation aus Chloroform/Methanol); [α]_{D}=+86 (CHCl₃; c=0,9); IR (CHCl₃): 3602 cm⁻¹.

### Beispiel 4

### 14α,17α-Propanoestra-1,3,5(10)-trien-3,17β-diol (18)

Zu einer Lösung von Verbindung **17** (105 mg; 0,32 mmol) in absolutem Toluol (5 ml) wird Diisobutylaluminiumhydrid (1 molar in Hexan; 3,2 ml; 3,2 mmol) hinzugegeben. Man kocht 24 Stunden unter Rückfluß. Nach Abkühlung auf Raumtemperatur wird mit verdünnter Salzsäure angesäuert und mit Methanol/Chloroform (1:9) extrahiert. Man engt am Vakuum ein und reinigt das Rohprodukt durch Säulenchromatographie an Kieselgel mit einem Gemisch aus Ethylacetat/Toluol (3:7). Man erhält 61 mg der Verbindung **18**.
Fp.=228-231°C (nach Kristallisation aus Ethylacetat: [α]²⁰_{D}=+87° (c=0,6; Tetrahydrofuran).

Das nachfolgende Reaktionsschema gibt die Herstellung der beispielgemäßen Verbindungen und der dazu benötigten Ausgangsverbindung wieder:

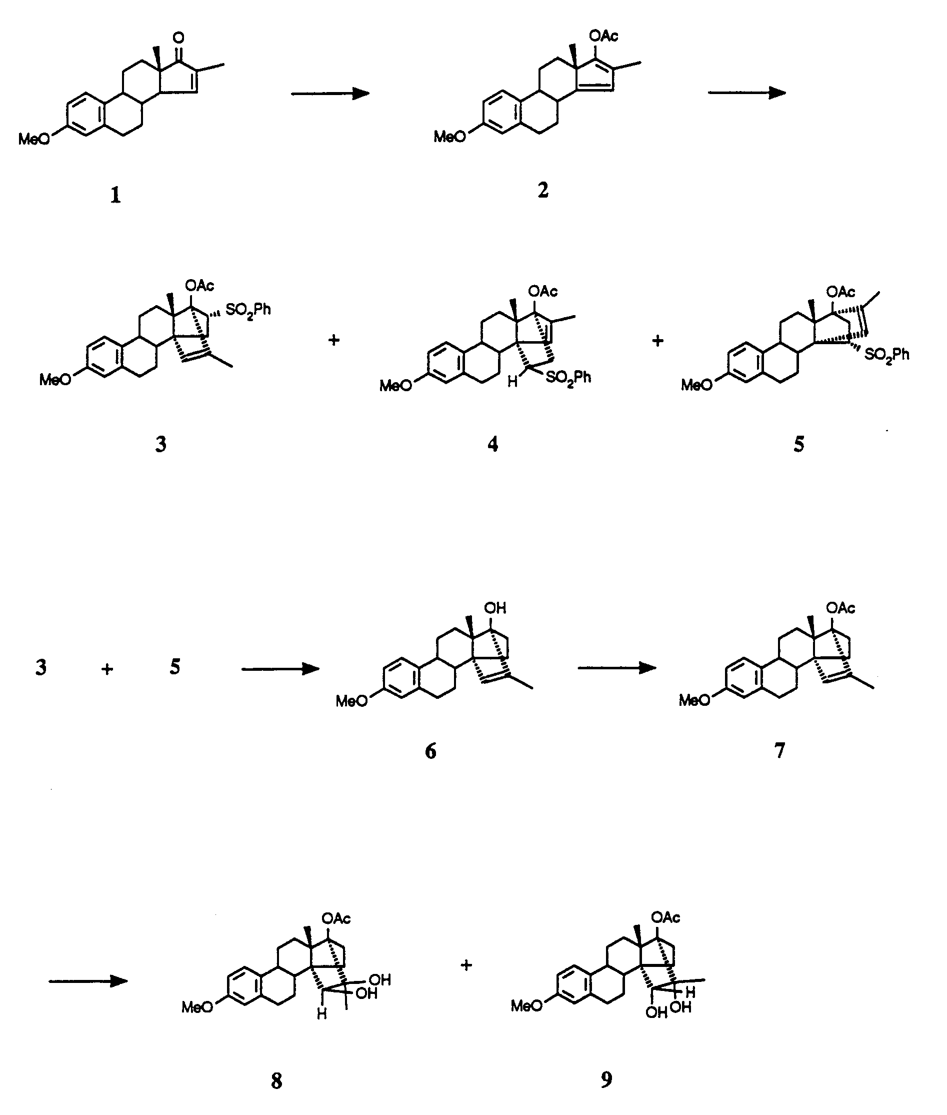

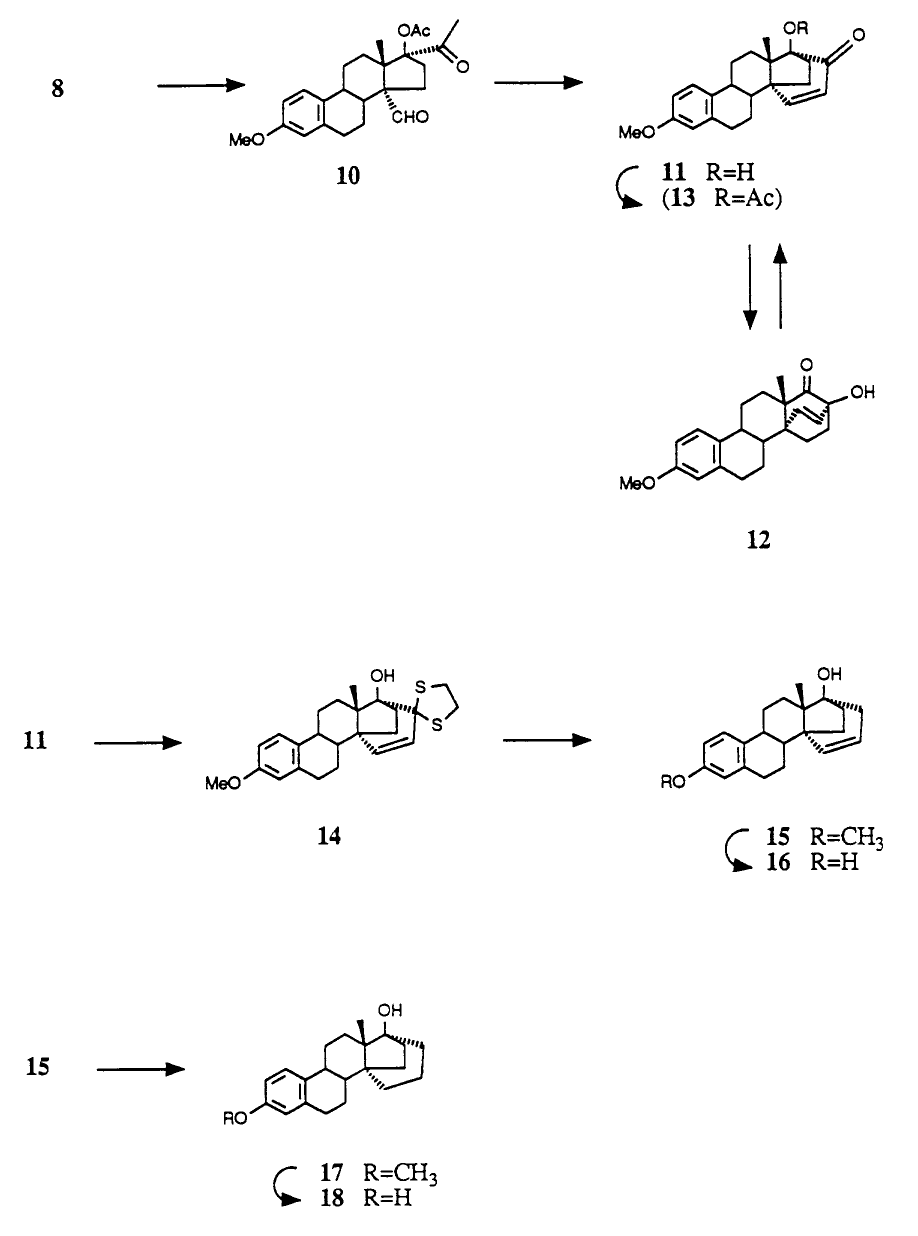

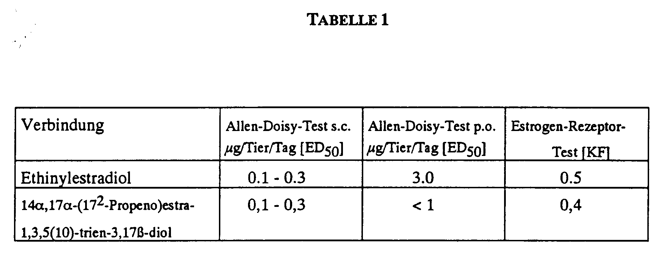

## Patentansprüche

1. 14α,17α-(Propano und 17²-Propeno)estratriene der allgemeinen Formel I 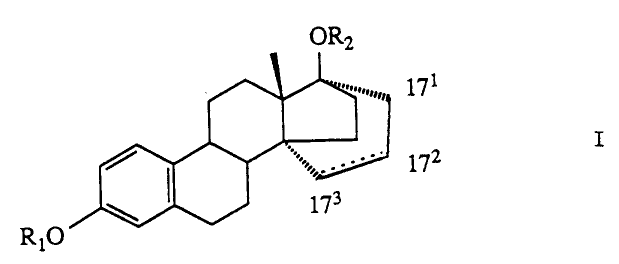 worin
R¹ ein Wasserstoffatom, eine Methyl- oder eine Acylgruppe mit 1-12 Kohlenstoffatomen,
R² ein Wasserstoffatom oder eine Acylgruppe mit 1-12 Kohlenstoffatomen und 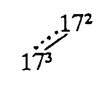 eine C-C-Einfach- oder Doppelbindung bedeuten.

2. Verbindungen gemäß Anspruch 1:
3-Methoxy-14α,17α-(17²-propeno)estra-1,3,5(10)-trien-17β-ol,
14α,17α-(17²-Propeno)estra-1,3,5(10)-trien-3,17β-diol,
3-Methoxy-14α,17α-(-propano)estra-1,3,5(10)-trien-17β-ol,
14α,17α-(-Propano)estra-1,3,5(10)-trien-3,17β-diol,

3. Östrogen wirksame Mittel, gekennzeichnet durch den Gehalt an einer Verbindung gemäß Anspruch 1 oder 2.

4. Verfahren zur Herstellung von 14α,17α-(Propano und 17²-Propeno)estratrienen der allgemeinen Formel I 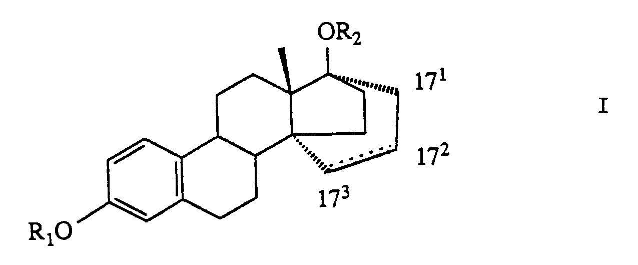 worin
R¹ ein Wasserstoffatom, eine Methyl- oder eine Acylgruppe mit 1-12 Kohlenstoffatomen,
R² ein Wasserstoffatom oder eine Acylgruppe mit 1-12 Kohlenstoffatomen und 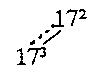 eine C-C-Einfach- oder Doppelbindung
bedeuten, dadurch gekennzeichnet, daß man im Hydroxyketon der Formel II 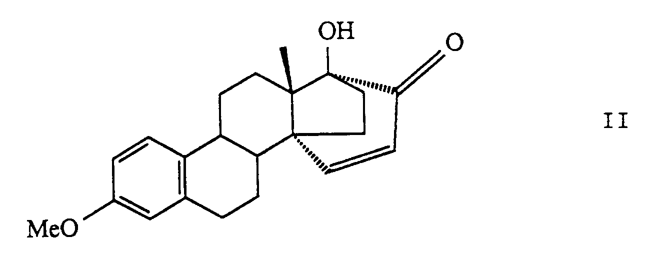 die Carbonylgruppe zur Methylengruppe reduziert, gegebenenfalls die 17²-17³-Doppelbindung hydriert, gegebenenfalls den 3-Methylether spaltet oder die 17-Hydroxygruppe verestert, gegebenenfalls die 3-Hydroxygruppe partiell oder die 3- und 17-Hydroxygruppen gleichzeitig verestert und gegebenenfalls eine so erhaltene 3,17-Diacyloxy- selektiv zur 3-Hydroxy-17-acyloxy-Verbindung verseift.

## Claims

1. 14α,17α-(propano and 17²-propeno)oestratrienes of the general formula I 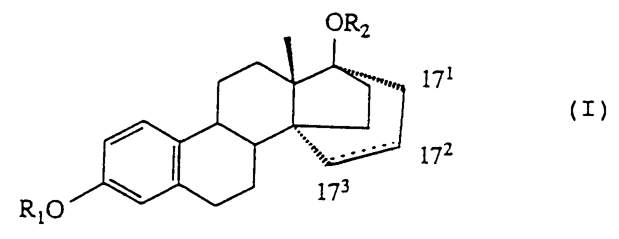 wherein
R¹ is a hydrogen atom, or a methyl group or an acyl group having from 1 to 12 carbon atoms,
R² is a hydrogen atom or an acyl group having from 1 to 12 carbon atoms, and 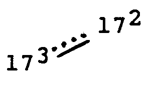
is a single or double C-C bond.

2. Compounds according to claim 1:
3-methoxy-14α,17α-(17²-propeno)oestra-1,3,5(10)-trien-17β-ol,
14α,17α-(17²-propeno)oestra-1,3,5(10)-triene-3,17β-diol,
3-methoxy-14α,17α-(propano)oestra-1,3,5(10)-trien-17β-ol,
14α,17α-(propano)oestra-1,3,5(10)-triene-3,17β-diol.

3. Oestrogenic preparations, characterised in that they contain a compound according to claim 1 or claim 2.

4. Process for the preparation of 14α,17α-(propano and 17²-propeno)oestratrienes of the general formula I 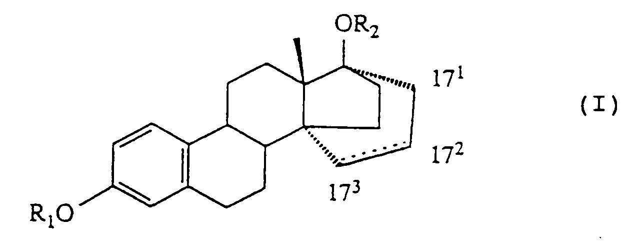 wherein
R¹ is a hydrogen atom, or a methyl group or an acyl group having from 1 to 12 carbon atoms,
R² is a hydrogen atom or an acyl group having from 1 to 12 carbon atoms, and 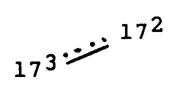
is a single or double C-C bond,
which process is characterised in that in the hydroxyketone of formula II 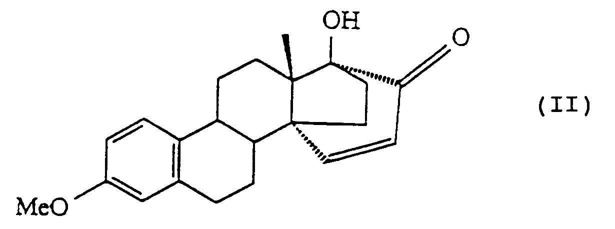 the carbonyl group is reduced to the methylene group, optionally the 17²-17³ double bond is hydrogenated, optionally the 3-methyl ether is cleaved or the 17-hydroxy group is esterified, optionally the 3-hydroxy group is partially esterified or the 3- and 17-hydroxy groups are simultaneously esterified and optionally a resulting 3,17-diacyloxy compound is hydrolysed selectively to the 3-hydroxy-17-acyloxy compound.

## Revendications

1. 14α,17α-(propano et 17²-propèno)estratriène de formule générale I 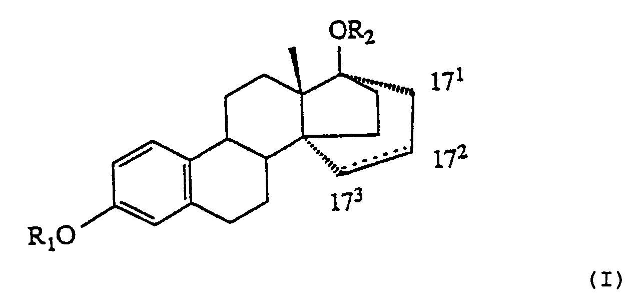 dans laquelle
R¹ signifie un atome d'hydrogène, un groupe méthyle ou un groupe acyle ayant de 1 à 12 atomes de carbone,
R² signifie un atome d'hydrogène ou un groupe acyle ayant de 1 à 12 atomes de carbone et 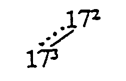 signifie une liaison simple carbone-carbone ou bien une liaison double.

2. Composés selon la revendication 1 :
3-méthoxy-14α,17α-(17²-propèno)estra-1,3,5(10)-triène-17β-ol,
14α,17α-(17²-propèno)estra-1,3,5(10)-triène-3,17β-diol,
3-méthoxy-14α,17α-(-propano)estra-1,3,5(10)-triène-17β-ol,
14α,17α-(-propano)estra-1,3,5(10)-triène-3,17β-diol.

3. Agent à activité oestrogène, caractérisé en ce qu'il contient un composé selon la revendication 1 ou 2.

4. Procédé de préparation de 14α,17α-(propano et 17²-propèno)estratriènes de formule générale I 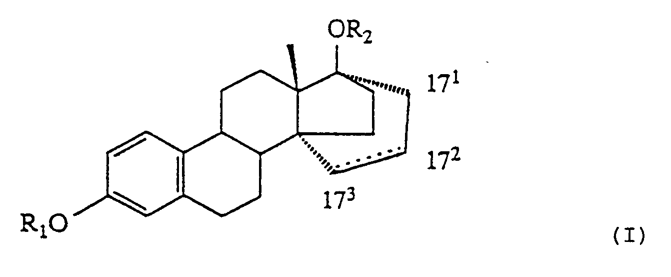 dans laquelle
R¹ signifie un atome d'hydrogène, un groupe méthyle ou un groupe acyle ayant de 1 à 12 atomes de carbone,
R² signifie un atome d'hydrogène ou bien un goupe acyle ayant de 1 à 12 atomes de carbone et 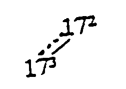 signifie une liaison simple carbone-carbone ou bien une liaison double,
caractérisé en ce qu'on réduit le groupe carbonyle en groupe méthylène dans l'hydroxy-cétone de formule II 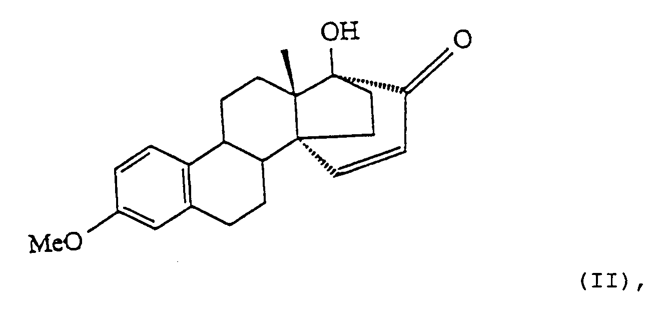 on hydrogène éventuellement la double liaison 17²-17³, on clive éventuellement l'éther méthylique en position 3 ou bien on estérifie le groupe hydroxy en position 17, on estérifie éventuellement partiellement le groupe hydroxy en position 3 ou bien simultanément les groupes hydroxy en positions 3 et 17 et on saponifie éventuellement de manière sélective un composé 3,17-diacyloxy ainsi obtenu en composé 3-hydroxy-17-acyloxy.
